# EUROPÄISCHE PATENTSCHRIFT

(11) **EP 3 218 039 B1**
(45) Veröffentlichungstag und Bekanntmachung des Hinweises auf die Patenterteilung: **25.07.2018**
(21) Anmeldenummer: 14808525.1
(22) Anmeldetag: 13.11.2014
(51) Int. Cl.: A61M 16/08, A61M 16/06

(54) **MULTIFUNKTIONALER, MOBIL EINSATZFÄHIGER APPLIKATOR**
MULTIFUNCTIONAL APPLICATOR WHICH CAN BE USED IN A MOBILE MANNER
APPLICATEUR MULTIFONCTIONNEL UTILISABLE DE MANIÈRE MOBILE

(43) Veröffentlichungstag der Anmeldung: 20.09.2017
(73) Patentinhaber: TNI medical AG, 97084 Würzburg (DE)
(72) Erfinder: ANGER, Ewald, 97246 Eibelstadt (DE); KLAUS, Dieter, 79689 Maulburg (DE); URBAN, Peter, 79117 Freiburg (DE)
(74) Vertreter: advotec.
(86) Internationale Anmeldenummer: PCT/EP2014/074457
(87) Internationale Veröffentlichungsnummer: WO 2016/074722

(56) Entgegenhaltungen:
- WO-A2-2008/060295
- DE-A1- 10 205 955
- DE-A1-102005 000 922
- US-A- 6 158 430
- US-A1- 2005 161 043

## Beschreibung

### Stand der Technik

Nasale Insufflationssysteme für die High Flow Therapie sind bekannt, beispielsweise aus der WO 2008/060 295 A2. Gefilterte Luft wird von einem Gebläse angesaugt, verdichtet, mit Sauerstoff angereichert, dieses Gemisch in einem beheizten Befeuchter vernebelt und dem Patienten mittels Applikator als Therapieluft in die Nasenlöcher zugeführt, sofern es sich bei dem Patienten um einen Erwachsenen handelt. Bei Kindern wird zusätzlich ein Mundstück benötigt.

Bei dem aus oben genannter Offenlegungsschrift bekannten nasalen Insufflationssystem wird die Anreicherung der Therapieluft mit Sauerstoff in einem High Flow Therapiegerät mittels eines mikroprozessorgesteuerten Ventils erreicht. Die durch einen Lufteinlass im High Flow Therapiegerät angesaugte und gefilterte Umgebungsluft wird dadurch mit reinem Sauerstoff, der über einen gesonderten Anschluss am High Flow Therapiegerät zugeführt wird, gezielt vermischt. Neben dem Sauerstoffgehalt steuert der Mikroprozessor noch weitere Parameter der zugeführten Therapieluft, wie Gasdruck, Gasfluss, Kohlendioxidgehalt, Temperatur und Feuchte. Der Applikator ist bei Verwendung des nasalen Insufflationssystems unmittelbar und dauerhaft mit dem High Flow Therapiegerät verbunden.

Aus der DE 10 2005 000 922 A1 ist ein Applikator bekannt, der einen Applikatorstecker, einen Zuleitungsschlauch, ein Y-Stück, Gabelschläuche und ein Nasenstück mit Zinken aufweist. Der Applikatorstecker seinerseits weist elektrische und pneumatische Steckerteile auf. Von einem derartigen Applikator wird bei der vorliegenden Erfindung ausgegangen.

In der DE 102 05 955 A1 ist ein Verfahren und eine Vorrichtung zur Bereitstellung von Atemgas offenbart, wobei ein Sauerstoffgenerator eine elektrolytische Zersetzung von Wasser in Wasserstoff und Sauerstoff durchführt und der erzeugte Sauerstoff komprimiert in eine Druckgasflasche eingespeist wird. Der Sauerstoffgenerator führt sowohl eine Befüllung der Druckgasflasche als auch eine Atemgasversorgung für eine Sauerstoffbrille mittels einer mit einem Verteiler versehenen Steuereinrichtung durch, der in Abhängigkeit von jeweiligen Steuerungsvorgaben eine Weiterleitung des Atemgases in Richtung auf die Sauerstoffbrille und/oder in Richtung der Druckgasflasche vorgibt. Ebenfalls offenbart ist, dass ein Patient wahlweise eine Verbindung über eine Verbrauchssteuerung als Anschlusselement an einen Sauerstoffgenerator oder an eine Druckgasflasche vornehmen kann. Damit einem Patienten ein relativ großer Bewegungsbereich in dessen Wohnung oder in einem anderen Aufenthaltsraum bereitgestellt wird, wird ein längerer Verbindungsschlauch zur Sauerstoffbrille vorgeschlagen.

Aus der DE 38 349 A ist eine Atmungsvorrichtung zum Eindringen in Räume mit nicht atembarer Atmosphäre bekannt, wobei über einen durch einen Handhebel verstellbaren Dreiwegehahn die Luftzufuhr aus einem festangeschlossenen, mitführbaren Sauerstoffbehälter zum Atmungsorgan zu- oder abgestellt werden kann und gleichzeitig ein Schlauch, über den ansonsten eine Außenluftversorgung erfolgt, abrespektive angekuppelt wird, was durch ein am Kücken des Dreiwegehahns befestigtes Kupplungsteil erfolgt.

Ein insbesondere für Krankenhäuser vorgesehenes Beatmungssystem zur Durchführung von CPAP- oder Sauerstofftherapien ist aus der US 6,158,430 A bekannt. Dieses besteht aus einem oder mehreren tragbaren Beatmungseinrichtungen, die mittels zugehöriger Schnittstellen an an unterschiedlichen Orten oder Stationen aufgestellten Dockingstationen betreibbar sind. Die Dockingstationen sind ihrerseits mit einer zentralen Gasversorgung des Krankenhauses verbunden, um die Sauerstoffzufuhr für die Beatmungseinrichtungen bereitzustellen. Ergänzend können auch einzelne Gasflaschen mit einer Dockingstation direkt verbunden werden.

Aus der WO 2013/089714 A1 ist eine hybride Selbstrettungsausrüstung bekannt, welche es Personen ermöglicht, sich in widrigen Umgebungen, beispielsweise Bergwerken, Tunneln oder Tanks mit kontaminierter Luft, zu bewegen. Dazu sind alle notwendigen Bauteile wie Atemschlauch, Mundstück, Atemmaske, Nasenklammern, Schutzbrille etc. in einem tragbaren Gehäuse untergebracht. Weiterhin weist die Selbstrettungsausrüstung ein Ventilgehäuse mit mehreren Anschlüssen auf, an die wahlweise Atemluftschläuche oder Staubfilter angeschlossen werden können.

Da Applikatoren, wie sie z.B. aus der DE 10 2005 000 922 A1 bekannt sind, immer zur Versorgung der Patienten mit dem High Flow Therapiegerät verbunden sein müssen und das High Flow Therapiegerät aufgrund seiner körperlichen Größe und Masse nicht beliebig transportierbar ist, ist der Patient an das High Flow Therapiegerät gebunden und somit immobil. Auch kann der Zuleitungsschlauch nicht beliebig lang ausgeführt werden, um dem Patienten etwas Bewegungsmöglichkeit einzuräumen, da die Parameter der zugeführten Therapieluft, besonders die Fluss- und Druckeigenschaften, nicht an dem Nasenstück mit den eingestellten Parametern sichergestellt werden können, wie sie das High Flow Therapiegerät erzeugt, da sich diese Parameter auf dem Weg vom High Flow Therapiegerät zum Nasenstück aufgrund von äußeren Einflüssen verändern.

Die nicht vorhandene Mobilität des Patienten ist von erheblichem Nachteil. Der Patient ist nicht in der Lage, kurzzeitig kleinere Wege ohne Gasversorgung und damit Atemunterstützung durchzuführen.

### Zugrunde liegendes Problem und Lösung

Diesen Mangel zu beseitigen, gleichzeitig ein Maximum an Mobilität und Flexibilität zu erzielen und weiterhin eine Versorgung mit Sauerstoff oder Therapieluft zu ermöglichen, liegt der vorliegenden Erfindung als Aufgabe zugrunde. Diese Aufgabe wird durch einen multifunktionalen, mobil einsatzfähigen Applikator nach dem vorliegenden Patentanspruch 1 gelöst. Weitere Vorteile der Erfindung geben die Merkmale der Unteransprüche an.

### Erreichte Vorteile

Der multifunktionale, mobil einsatzfähige Applikator ermöglicht mit seiner vorteilhaften Konstruktion des Applikatorsteckers einerseits die kontinuierliche Versorgung mit der vom High Flow Therapiegerät bereitgestellten konditionierten Therapieluft (TNI) und gewährleistet andererseits die Versorgung mit Sauerstoff (LTOT) oder mit Therapieluft, wenn der Patient den Applikatorstecker vom High Flow Therapiegerät abnimmt, beispielsweise um die Toilette, Küche etc. aufzusuchen, bei automatischer Umschaltung.

Bei diesen Gängen wird der Patient von einer leicht zu handhabenden tragbaren Sauerstoff- oder Therapieluftquelle versorgt und somit ist die Sauerstoff- bzw. Therapieluft-Unterstützung ununterbrochen sichergestellt. Dies ist für den Patienten ein erheblicher Gewinn an Lebensqualität, er braucht ein Abnehmen oder Versagen seiner Atmungsunterstützung nicht zu befürchten und ist von daher vor einem möglichen lebensbedrohlichen Zustand geschützt.

Darüber hinaus soll der Patient bei aufgestecktem Applikator in jedem seiner Nasenlöcher mit einem konstanten Volumenfluss der Therapieluft versorgt werden, da nur so der Totraum zuverlässig ausgespült wird. Dazu werden die Parameter der Therapieluft nahe beim Patienten gemessen und diese vorrangig im High Flow Therapiegerät so geregelt, dass der Volumenfluss konstant an den Austrittsöffnungen der Zinken der Nasenbrille gehalten wird. Dies wird durch vorteilhafte, in der Beschreibung näher erläuterte Maßnahmen erreicht.

Die Ausgestaltung des Ventilkörpers als Kugel nach Patentanspruch 2 ermöglicht einen einfachen Aufbau mit den Ventilsitzen und ihren Dichtungen, um die Ventilfunktion sicherzustellen.

Wird dagegen der Ventilkörper als Zylinderkolben ausgestaltet, wie im Patentanspruch 3 beansprucht, werden die bei dieser Ausführungsform üblichen Vorteile, wie z. B. bessere und leichtere Abdichtungen, erzielt.

Da die im Applikator befindlichen elektronischen Bauteile mit Energie versorgt werden müssen, sind nach Patentanspruch 4 Kontakte für eine Stromversorgung und/oder für Signal- und Datenleitungen bereits im Applikatorstecker vorhanden, und es werden keine zusätzlichen externen Verbindungsleitungen benötigt.

Nach Patentanspruch 5 ist im Y-Stück in vorteilhafter Weise eine Funktionseinheit eingebaut, die ein Wegeventil (Patentanspruch 6), Sender und Empfänger (Patentanspruch 7)und eine elektronische Verarbeitungseinheit (Patentanspruch 8) aufweist. Diese Bauteile werden in einfacher Weise über Heizdrähte im Zuleitungsschlauch oder Batterien oder Akkus mit Energie versorgt, wie die alternativ genannten Merkmale im Patentanspruch 9 aufzeigen.

Der Applikatorstecker selbst kann nach Patentanspruch 10 mit einem programmierbaren Mikroprozessor ausgestattet sein, dessen Speicher nach Patentanspruch 11 Patientendaten sowie Parameterangaben der jeweils individuell benötigten Therapieluft und Servicedaten enthält, die für die Auswertung durch die Steuerfunktion im High Flow Therapiegerät benötigt werden. Bei einem Auswechseln der Therapiegeräte kann somit der Patient ohne Neueinstellungen am Gerät mit gleichbleibender, ihm zugehöriger Therapieluft versorgt werden, da die Einstellwerte vom Applikatorstecker übernommen werden können.

Um individueller auf anatomische Asymmetrien reagieren zu können, ist das Y-Stück nicht näher als 5 mm von den Zinken angeordnet, wie Patentanspruch 12 festlegt.

Die in Patentanspruch 13 beanspruchte Verwendung von antibakteriell beschichteten Werkstoffen schützt den Patienten vor Infektionen.

Weiterhin ist von Vorteil, wie aus Patentanspruch 14 ersichtlich, dass am Sauerstoffanschluss statt Sauerstoff auch Therapieluft zugeführt werden kann.

### Beschreibung eines Ausführungsbeispiels

Es zeigen:
- Figur 1: ein nasales Insufflationssystem nach dem Stand der Technik,
- Figur 2: einen mit einem High Flow Therapiegerät verbundenen Applikatorstecker nach der Erfindung,
- Figur 3: ein Oberteil des erfindungsgemäßen Applikatorsteckers,
- Figur 4: einen vertikalen Schnitt durch den erfindungsgemäßen Applikatorstecker,
- Figur 5: einen vertikalen Schnitt durch den erfindungsgemäßen Applikatorstecker mit Betätigungselement in der Ebene des Sauerstoffanschlusses,
- Figur 6: einen vertikalen Schnitt durch das Oberteil des erfindungsgemäßen Applikatorsteckers in der Ebene des Therapieluftanschlusses,
- Figur 7: eine Bodenansicht des Applikatorsteckers nach der Erfindung,
- Figur 8: einen erfindungsgemäßen Applikator mit Funktionseinheit,
- Figur 9: Details der Funktionseinheit.

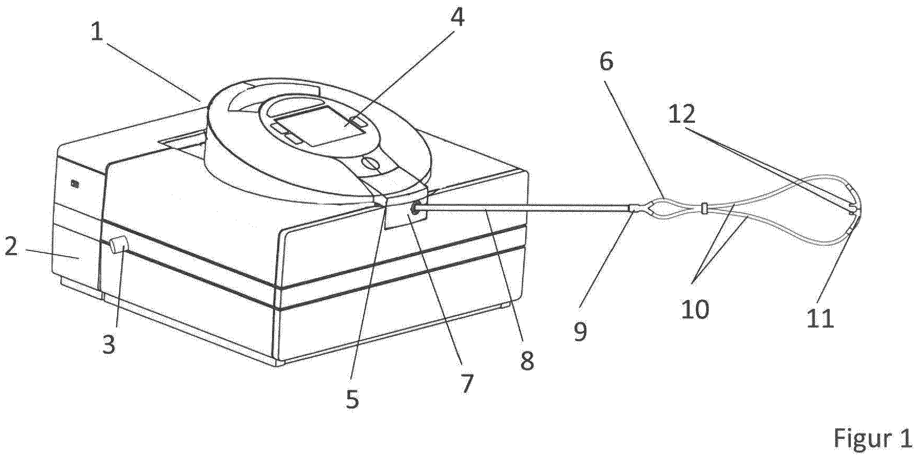

Figur 1 zeigt ein nasales Insufflationssystem bestehend aus einem High Flow Therapiegerät 1 und einem Applikator 6 nach dem Stand der Technik. Am High Flow Therapiegerät 1 sind der Einlass für die Umgebungsluft 2, der Anschluss für die Sauerstoffzufuhr 3, die Bedien- und Anzeigeeinrichtung 4 und am Ausgang für die Therapieluft der Anschluss 5 für den Applikator 6 erkennbar. Der Applikator 6 besteht grundsätzlich zumindest aus einem Applikatorstecker 7, einem Zuleitungsschlauch 8, einem Y-Stück 9, Gabelschläuchen 10 und einem Nasenstück 11 mit Zinken 12.

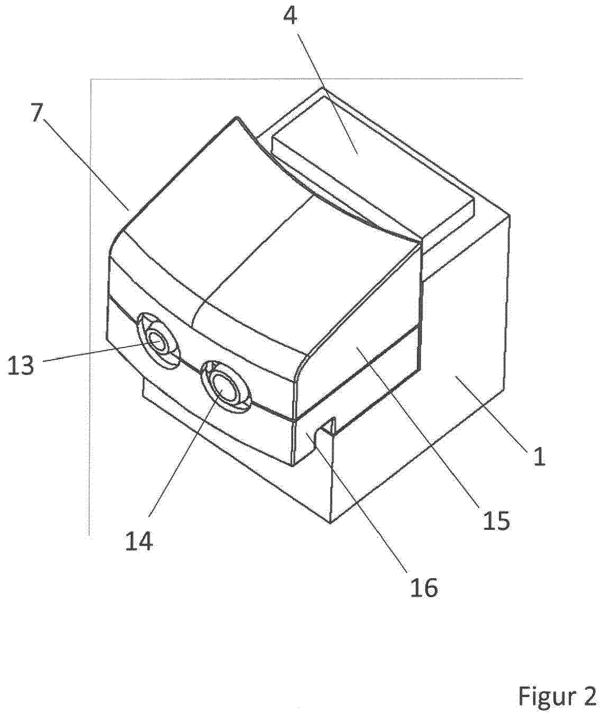

Der erfindungsgemäße multifunktionale, mobil einsatzfähige Applikator 6 ist mit einem Applikatorstecker 7 ausgestattet, der, wie Figur 2 zeigt, auf dem High Flow Therapiegerät 1 anbringbar ist, und besitzt einen Sauerstoffanschluss 13 und einen Therapieluftanschluss 14 für den in dieser Figur nicht gezeigten Zuleitungsschlauch 8. Der Applikatorstecker 7 besteht mindestens aus einem Oberteil 15 und einem Bodenteil 16.

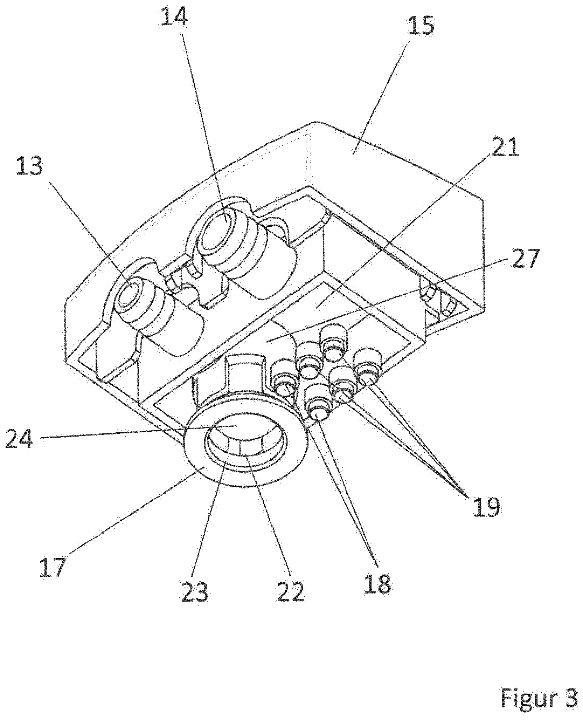

Figur 3 zeigt das Oberteil 15 des Applikatorsteckers 7 von unten in teilweiser Ansicht mit einer Befeuchterschnittstelle 17, durch welche der Applikatorstecker 7 mit konditionierter Therapieluft vom High Flow Therapiegerät 1 versorgt wird. Dargestellt ist weiterhin ein unterer Ventilsitz 23 mit seiner Dichtung 22 und darüber ein Ventilkörper 24, welcher in einer Führung 27 geradlinig bewegbar ist. Diese ist in einer Druckkammer 21 angeordnet. Daneben sind Kontakte 18/19 für die Stromversorgung und für Signal- und/oder Datenleitungen dargestellt.

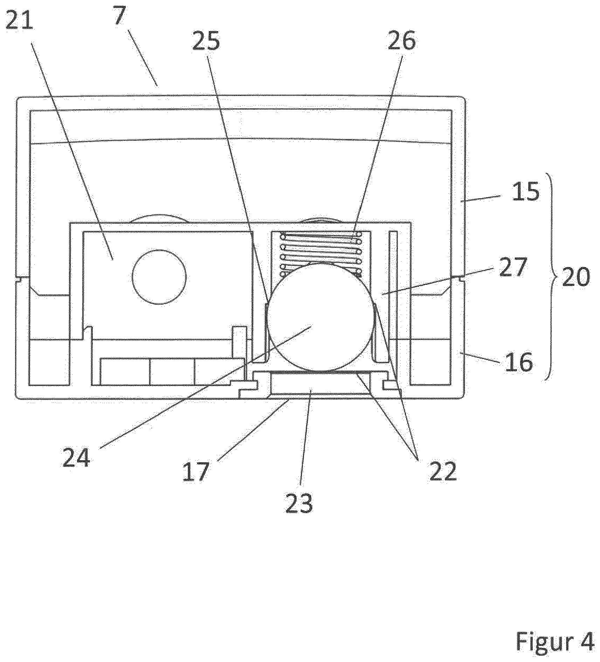

Ein vertikaler Schnitt durch den Applikatorstecker 7, Figur 4, zeigt sein aus Oberteil 15 und Bodenteil 16 bestehendes Gehäuse 20 mit der Druckkammer 21, in die von unten aus dem High Flow Therapiegerät 1 über die Befeuchterschnittstelle 17 Therapieluft einströmen kann. Diese Befeuchterschnittstelle 17 besitzt an ihrem oberen Ende den mit der Dichtung 22 ausgestatteten unteren Ventilsitz 23, auf welchem der Ventilkörper 24 (z.B. wie hier eine Kugel oder ein zylindrischer Ventilkolben) ruht, wenn der Applikatorstecker 7 nicht auf dem High Flow Therapiegerät 1 sitzt. Oberhalb des Ventilkörpers 24 befindet sich ein weiterer, ebenfalls mit einer Dichtung 22 ausgestatteter oberer Ventilsitz 25, der eine Verbindung mit dem Sauerstoffanschluss 13 herstellen kann. Auch ist eine Schraubendruckfeder 26 sichtbar, welche mit ihrer Federkraft auf den Ventilkörper 24 einwirkt und diesen so auf die Dichtung 22 des unteren Ventilsitzes 23 der Befeuchterschnittstelle 17 drückt. So wird, wenn der Applikatorstecker 7 vom High Flow Therapiegerät 1 abgenommen ist, die Befeuchterschnittstelle 17 zuverlässig abgedichtet.

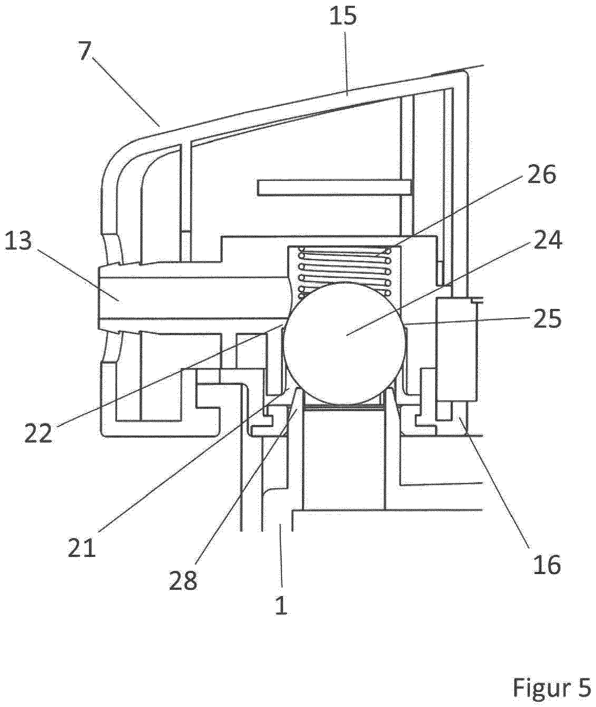

Der Ventilkörper 24 wird, wenn der Applikationsstecker 7 auf dem High Flow Therapiegerät 1 mittels Verrastung oder Arretierung fest sitzt, durch ein an diesem ortsfest angebrachtes Betätigungselement 28, wie Figur 5 zeigt, nach oben gegen die Dichtung 22 des oberen Ventilsitzes 25 gedrückt. Somit kann kein Sauerstoff über den Sauerstoffanschluss 13 in die Druckkammer 21 einströmen. Dies stellt sicher, dass die vom High Flow Therapiegerät 1 bereitgestellte Therapieluft in die Druckkammer 21 hinein und weiter über den, hier nicht in dieser Schnittebene darstellbaren, Therapieluftanschluss 14 zum Zuleitungsschlauch 8 strömt. Dieser Strömungsweg ist der Regelfall.

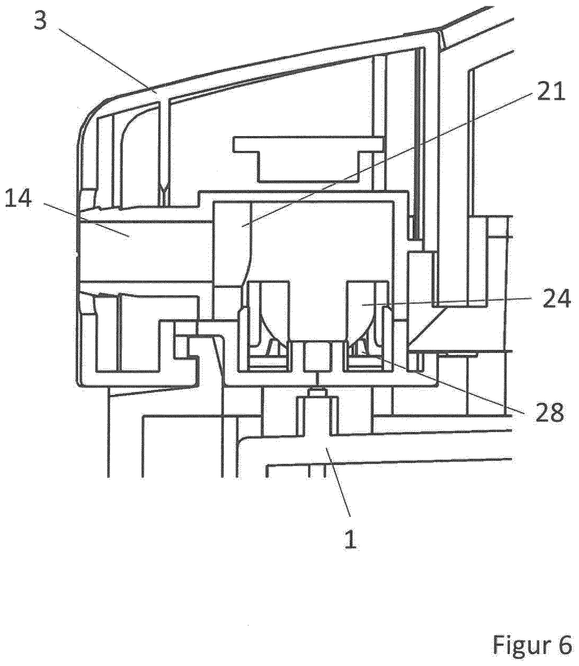

Einen vertikalen Schnitt durch den Applikatorstecker 7 des multifunktionalen, mobil einsatzfähigen Applikators 6 in der Ebene des Therapieluftanschlusses 14 für den, hier nicht dargestellten, Zuleitungsschlauch 8 zeigt Figur 6. Dieser Therapieluftanschluss 14 stellt die Verbindung der Druckkammer 21 mit dem Zuleitungsschlauch 8 her. Diese Verbindung ist bei aufgesetztem, fest verrastetem oder arretiertem Applikatorstecker 7 auf dem High Flow Therapiegerät 1 immer offen, da das Betätigungselement 28 den Ventilköper 24 von der in Figur 5 gezeigten Dichtung 22 des Ventilsitzes 23 abhebt und die Druckkammer 21 dann immer mit Therapieluft versorgt.

Besteht der Wunsch des Patienten, für einen abschätzbaren Zeitraum mobil zu sein, ist die tragbare und auf ihren Füllungsgrad überwachte Sauerstoffflasche spätestens dann an den Sauerstoffanschluss 13 anzuschließen, falls dies nicht schon erfolgte. Wird der Applikatorstecker 7 nun von dem High Flow Therapiegerät 1 abgenommen, wirkt das Betätigungselement 28 nicht mehr auf den Ventilkörper 24. Durch die Kraft der Schraubendruckfeder 26 wird der Ventilkörper 24 auf die Dichtung 22 des unteren Ventilsitzes 23 gepresst und verschließt diesen. Sauerstoff strömt dann über den Sauerstoffanschluss 13 in die Druckkammer 21 ein, um weiter über den Therapieluftanschluss 14 in den Zuleitungsschlauch 8 zu gelangen. Für den Patienten ist der multifunktionale, mobil einsatzfähige Applikator 6 mit einer kleinen, leicht zu handhabenden Sauerstoff- oder Therapieluftquelle ohne Aufwand und bequem bei seiner Bewegung zu handhaben und ermöglicht erst dadurch die mobile Bewegungsmöglichkeit unter Beibehaltung dieser Sauerstoff-bzw. Therapieluft-Versorgung ohne das High Flow Therapiegerät 1. Die ununterbrochene Versorgung mit Sauerstoff- bzw. Therapieluft wird so auch während der Bewegung zuverlässig sichergestellt, was ohne den vorstehend beschriebenen, erfindungsgemäßen Ventilaufbau und seine Funktion nicht möglich ist.

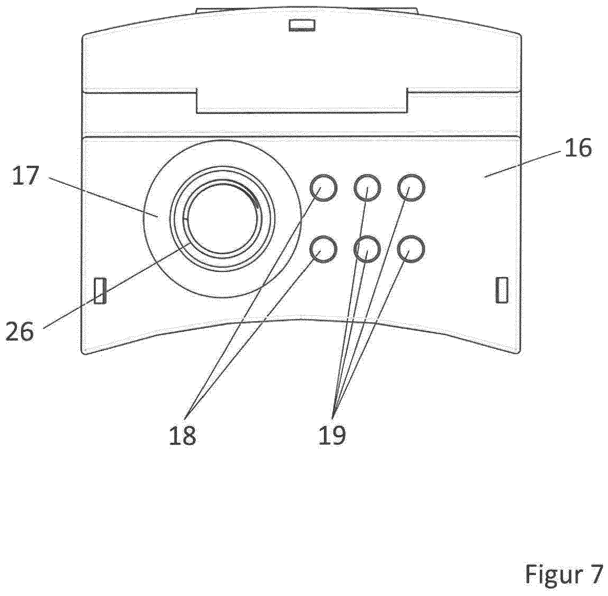

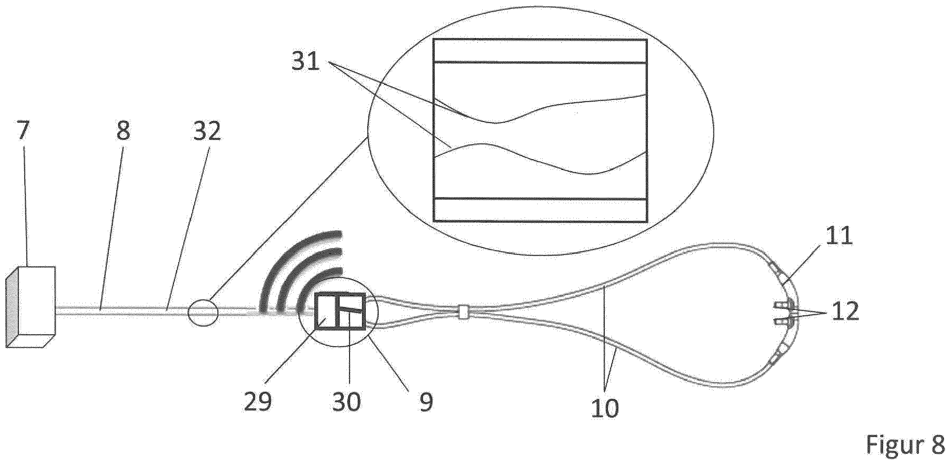

Die Ansicht des Applikatorsteckers 7 von unten (Aufsteckseite zum High Flow Gerät) in Figur 7 zeigt neben der Befeuchterschnittstelle 17, da der Ventilkörper 24 hier nicht dargestellt ist, die Schraubendruckfeder 26 sowie die Kontakte 18 und 19 für die Stromversorgung und/oder die Signal- und/oder Datenleitungen (z.B. für Datenleitung 32, Figur 8). Diese Kontakte korrespondieren mit entsprechenden Kontakten des High Flow Therapiegeräts 1.

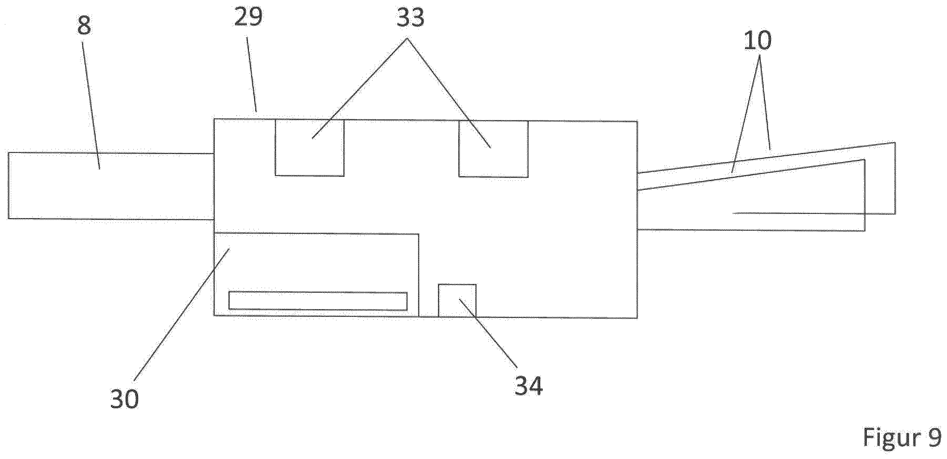

Neben der Funktion der mobilen Einsatzfähigkeit ermöglicht der erfindungsgemäße multifunktionale, mobil einsatzfähige Applikator 6 noch Überwachungsfunktionen (wie z. B. Nutzungszeit, Temperatur, Serviceintervalle usw.), wie sie im Hinblick auf die Figuren 8 und 9 näher beschrieben werden.

Bei Bedarf kann das Y-Stück 9, welches die Therapieluft durchleitet, mit einer Funktionseinheit 29 ausgestattet werden, wie Figur 8 zeigt. Diese Funktionseinheit 29 enthält Sensoren 33 (Figur 9), welche z.B. Volumenfluss, Feuchte, Temperatur etc. messen. Die gemessenen Daten werden mittels einer Datenleitung 32 im Zuleitungsschlauch 8 zum Applikatorstecker 7 und weiter über die Kontakte 19 zum High Flow Therapiegerät 1 gegeben. Alternativ zur Datenleitung 32 verfügt die Funktionseinheit 29 über Sender und Empfänger zur Datenübertragung oder überträgt diese Daten direkt über die Heizleitung. Diese Sender und Empfänger kommunizieren mit dem entsprechenden Empfänger und Sender entweder im Applikatorstecker 7 oder im High Flow Therapiegerät 1. Wenn Sender und Empfänger im Applikatorstecker 7 eingebaut sind, werden die Daten mittels der Kontakte 19 dem High Flow Therapiegerät 1 weitergeleitet, damit dieses die Parameter der Therapieluft optimieren kann. Die Energieversorgung der Sender und Empfänger in der Funktionseinheit 29 kann durch Batterien oder Akkus erfolgen. Möglich ist auch die Energieversorgung mittels der im Zuleitungsschlauch 8 verlaufenden Heizdrähte 31, welche die notwendige Energie mit übertragen. Auch können die Akkus mittels der Heizdrähte 31 geladen werden (siehe vergrößerten Ausschnitt in Figur 8).

Um diese Funktionen auszuführen, verfügt die Funktionseinheit 29, wie Figur 9 zeigt, über Sensoren 33 zum Messen der Parameter der vorbei strömenden Therapieluft und eine elektronische Verarbeitungseinheit 34, welche die gemessenen Daten weitergibt.

Neben den genannten Sensoren 33 kann in der Funktionseinheit 29 ein Wegeventil 30 vorgesehen sein, welches die Therapieluft unterschiedlich zu den Gabelschläuchen 10 leitet. Damit wird über die Gabelschläuche 10 alternativ in Abhängigkeit von geeigneten Sensoren im Bereich der Zinken 12 an deren Austrittsöffnungen der konstante Volumenfluss sicher gestellt. Der unterschiedliche Widerstand der Atemwege des Patienten durch die Nasenlöcher wird so berücksichtigt.

Der Applikatorstecker 7 kann weiterhin einen programmierbaren Mikroprozessor aufweisen, in welchem Servicedaten, die Daten und Parameter des Patienten, der den multifunktionalen, mobil einsatzfähigen Applikator 6 benutzt, abgelegt sind. Diese Daten können für die individuelle Steuerung des High Flow Therapiegeräts 1 notwendig sein, da so die korrekte Konditionierung der Therapieluft eingestellt werden kann. Wechselt der Patient mit seinem multifunktionalen, mobil einsatzfähigen Applikator 6 das bisher benutzte High Flow Therapiegerät 1, so stellt sich dieses automatisch um und der Patient erhält sofort eine konditionierte Therapieluft mit den für ihn notwendigen und definierten Parametern, er ist also in dieser Beziehung von dem gewählten High Flow Therapiegerät 1 unabhängig. Dies setzt voraus, dass das High Flow Therapiegerät 1 seinerseits über eine entsprechende Steuerung verfügt, welche die Daten aus dem Mikroprozessor im Applikationsstecker 7 verarbeiten kann.

Zu den Daten des Patienten zählen beispielsweise auch die applikatorspezifischen Eigenschaften, wie Flussbegrenzung bei einem Kinder-applikator, Identifikation des Applikatortyps, Betriebsstunden und Laufzeiten etc..

Alternativ zu dem Wegeventil 30 in der Funktionseinheit 29 kann sich im Nasenstück 11 zwischen den Zinken 12 ein angesteuertes Wegeventil befinden, um den Volumenfluss an den jeweiligen Austrittsöffnungen der Zinken 12 konstant zu halten. Diese Ansteuerung erfolgt mit der gleichen technischen Lösung, wie in der Funktionseinheit 29. Auch können die Austrittsquerschnitte der Zinken 12 asymmetrisch ausgebildet sein. So sind des Weiteren jeweils unterschiedliche Querschnittsflächen und/oder Querschnittsformen der Austrittsöffnungen der Zinken 12 im Nasenloch denkbar.

Der Innendurchmesser des Sauerstoffanschlusses 13 beträgt dabei mindestens 1 mm. Vorzugsweise liegt der Innendurchmesser des Sauerstoffanschlusses 13 zwischen 1 und 10 mm

Der Innendurchmesser des Therapieluftanschlusses 14 beträgt mindestens 2 mm. Vorzugsweise liegt der Innendurchmesser des Therapieluftanschlusses 14 zwischen 2 und 25 mm.

Ziel aller vorstehend beschriebenen Maßnahmen ist es, an den jeweiligen Austrittsöffnungen der Zinken 12 einen konstanten Volumenfluss unter Berücksichtigung aller dafür notwendigen Parameter bereitzustellen.

### Bezugszeichenliste

- 1: High Flow Therapiegerät
- 2: Einlass für Umgebungsluft
- 3: Anschluss für Sauerstoffzufuhr
- 4: Bedien- und Anzeigeteil des High Flow Therapiegeräts
- 5: Anschluss für Applikatorstecker
- 6: Applikator
- 7: Applikatorstecker
- 8: Zuleitungsschlauch
- 9: Y-Stück
- 10: Gabelschläuche
- 11: Nasenstück
- 12: Zinken
- 13: Sauerstoffanschluss
- 14: Therapieluftanschluss
- 15: Oberteil des Applikatorsteckers
- 16: Bodenteil des Applikatorsteckers
- 17: Befeuchterschnittstelle
- 18: Kontakte für Stromversorgung
- 19: Kontakte für Signal- und/oder Datenleitungen
- 20: Gehäuse des Applikatorsteckers
- 21: Druckkammer
- 22: Dichtung
- 23: unterer Ventilsitz
- 24: Ventilkörper
- 25: oberer Ventilsitz
- 26: Schraubendruckfeder
- 27: Führung für Ventilkörper
- 28: Betätigungselement
- 29: Funktionseinheit
- 30: Wegeventil
- 31: Heizdrähte
- 32: Datenleitung
- 33: Sensoren
- 34: elektronische Verarbeitungseinheit

## Patentansprüche

1. Multifunktionaler, mobil einsatzfähiger Applikator 6 aufweisend einen Applikatorstecker 7, einen Zuleitungsschlauch 8, ein Y-Stück 9, Gabelschläuche 10 und ein Nasenstück 11 mit Zinken 12,
**dadurch gekennzeichnet,**
**dass** der Applikatorstecker 7 eine Druckkammer 21 enthält, die eine Befeuchterschnittstelle 17 zur Verbindung mit einem High Flow Therapiegerät 1, einen Sauerstoffanschluss 13 mit einem Öffnungsdurchmesser von mindestens 1 mm und einen Therapieluftanschluss 14 für den Zuleitungsschlauch 8 aufweist, wobei die Befeuchterschnittstelle 17 und der Sauerstoffanschluss 13 innerhalb der Druckkammer 21 jeweils einen mit einer Dichtung 22 versehenen unteren und oberen Ventilsitz 23, 25 aufweisen und zwischen diesen Ventilsitzen 23, 25 ein Ventilkörper 24 bewegbar ist und dieser Ventilkörper 24 aus Richtung des Sauerstoffanschlusses 13 von einer Schraubendruckfeder 26 kraftbeaufschlagt wird, andererseits von einem Betätigungselement 28 des High Flow Therapiegeräts 1 gegen den oberen Ventilsitz 25 gedrückt wird, wenn der Applikatorstecker 7 auf dem High Flow Therapiegerät 1 arretiert sitzt.

2. Multifunktionaler, mobil einsatzfähiger Applikator 6 nach Patentanspruch 1,
**dadurch gekennzeichnet,**
**dass** der Ventilkörper 24 eine Kugel ist.

3. Multifunktionaler, mobil einsatzfähiger Applikator 6 nach Patentanspruch 1,
**dadurch gekennzeichnet,**
**dass** der Ventilkörper 24 ein Zylinderkolben ist.

4. Multifunktionaler, mobil einsatzfähiger Applikator 6 nach Patentanspruch 1, 2 oder 3,
**dadurch gekennzeichnet,**
**dass** der Applikatortecker 7 Kontakte 18, 19 für eine Stromversorgung und/oder für Signal- und/oder Datenleitungen aufweist.

5. Multifunktionaler, mobil einsatzfähiger Applikator 6 nach einem der vorhergehenden Patentansprüche,
**dadurch gekennzeichnet,**
**dass** im Y-Stück 9 eine Funktionseinheit 29 integriert ist, welche Sensoren 33 enthält.

6. Multifunktionaler, mobil einsatzfähiger Applikator 6 nach einem der vorhergehenden Patentansprüche,
**dadurch gekennzeichnet,**
**dass** im Y-Stück 9 eine Funktionseinheit 29 integriert ist, welche ein Wegeventil 30 aufweist.

7. Multifunktionaler, mobil einsatzfähiger Applikator 6 nach einem der vorhergehenden Patentansprüche,
**dadurch gekennzeichnet,**
**dass** im Y-Stück 9 eine Funktionseinheit 29 integriert ist, welche Sender und Empfänger aufweist, die entweder mit Sendern und Empfängern im Applikatorstecker 7 oder im High Flow Therapiegerät 1 kommunizieren.

8. Multifunktionaler, mobil einsatzfähiger Applikator 6 nach einem der vorhergehenden Patentansprüche,
**dadurch gekennzeichnet,**
**dass** im Y-Stück 9 eine Funktionseinheit 29 integriert ist, welche eine elektronische Verarbeitungseinheit 34 aufweist.

9. Multifunktionaler, mobil einsatzfähiger Applikator 6 nach Patentanspruch 8,
**dadurch gekennzeichnet,**
**dass** die elektronische Verarbeitungseinheit 34 über Heizdrähte 31 im Zuleitungsschlauch 8 oder durch eine Batterie oder einen Akku mit Energie versorgt wird.

10. Multifunktionaler, mobil einsatzfähiger Applikator 6 nach einem der vorhergehenden Patentansprüche,
**dadurch gekennzeichnet,**
**dass** der Applikatorstecker 7 einen programmierbaren Mikroprozessor aufweist.

11. Multifunktionaler, mobil einsatzfähiger Applikator 6 nach Patentanspruch 10,
**dadurch gekennzeichnet,**
**dass** im Speicher des programmierbaren Mikroprozessors Servicedaten, Daten des Patienten und seiner individuell benötigten Therapieparameter abgelegt sind.

12. Multifunktionaler, mobil einsatzfähiger Applikator 6 nach einem der vorhergehenden Patentansprüche,
**dadurch gekennzeichnet,**
**dass** das Y-Stück 9 nicht näher als 5 mm von den Zinken 12 angeordnet ist.

13. Multifunktionaler, mobil einsatzfähiger Applikator 6 nach einem der vorhergehenden Patentansprüche,
**dadurch gekennzeichnet,**
**dass** die verwendeten Werkstoffe antibakteriell beschichtet sind.

14. Multifunktionaler, mobil einsatzfähiger Applikator 6 nach einem der vorhergehenden Patentansprüche,
**dadurch gekennzeichnet,**
**dass** im mobilen Einsatz eine kleine, leicht zu handhabende Sauerstoff- oder Therapieluftquelle mit dem Sauerstoffanschluss 13 verbunden ist.

## Claims

1. A multifunctional applicator 6 for mobile use having an applicator plug 7, a supply tube 8, a Y-piece 9, fork tubes 10 and a nose piece 11 with prongs 12,
**characterised in that**
the applicator plug 7 comprises a pressure chamber 21 that has a humidifier interface 17 for connection with a high flow therapy device 1, an oxygen supply port 13 having an opening diameter of at least 1 mm, and a therapy air supply port 14 for the supply tube 8, the humidifier interface 17 and the oxygen supply port 13 within the pressure chamber 21 each having an upper and a lower valve seat 23, 25 that are provided with a seal 22 and a valve body 24 being movable between said valve seats 23, 25 and said valve body 24 being subjected to a force by a helical compression spring 26 from the direction of the oxygen supply port 13 and on the other hand being pushed against the upper valve seat 25 by an actuating element 28 of the high flow therapy device 1 when the applicator plug 7 is locked in place on the high flow therapy device 1.

2. The multifunctional applicator 6 for mobile use according to patent claim 1,
**characterised in that**
the valve body 24 is a sphere.

3. The multifunctional applicator 6 for mobile use according to patent claim 1,
**characterised in that**
the valve body 24 is a cylinder piston.

4. The multifunctional applicator 6 for mobile use according to patent claims 1, 2 or 3,
**characterised in that**
the applicator plug 7 has contacts 18, 19 for a power supply and/or for signal and/or data lines.

5. The multifunctional applicator 6 for mobile use according to any one of the preceding patent claims,
**characterised in that**
in the Y-piece 9, a function unit 29 is integrated that comprises sensors 33.

6. The multifunctional applicator 6 for mobile use according to any one of the preceding patent claims,
**characterised in that**
in the Y-piece 9, a function unit 29 is integrated that has a directional valve 30.

7. The multifunctional applicator 6 for mobile use according to any one of the preceding patent claims,
**characterised in that**
in the Y-piece 9, a function unit 29 is integrated that has transmitters and receivers that communicate with transmitters and receivers either in the applicator plug 7 or in the high flow therapy device 1.

8. The multifunctional applicator 6 for mobile use according to any one of the preceding patent claims,
**characterised in that**
in the Y-piece 9, a function unit 29 is integrated that has an electronic processing unit 34.

9. The multifunctional applicator 6 for mobile use according to patent claim 8,
**characterised in that**
the electronic processing unit 34 is supplied with energy via heating wires 31 in the supply tube 8 or by a battery or a rechargeable battery.

10. The multifunctional applicator 6 for mobile use according to any one of the preceding patent claims,
**characterised in that**
the applicator plug 7 has a programmable microprocessor.

11. The multifunctional applicator 6 for mobile use according to patent claim 10,
**characterised in that**
service data, patient data and therapy parameters individually required by the patient are stored in the memory of the programmable microprocessor.

12. The multifunctional applicator 6 for mobile use according to any one of the preceding patent claims,
**characterised in that**
the Y-piece 9 is arranged no closer than 5 mm to the prongs 12.

13. The multifunctional applicator 6 for mobile use according to any one of the preceding patent claims,
**characterised in that**
the materials used have an antibacterial coating.

14. The multifunctional applicator 6 for mobile use according to any one of the preceding patent claims,
**characterised in that**
in mobile use, a small, easy-to-handle oxygen or therapy air source is connected to the oxygen supply port 13.

## Revendications

1. Applicateur multifonctionnel 6 utilisable de manière mobile, comportant un connecteur d'applicateur 7, un tuyau d'alimentation 8, un raccord en Y 9, des tuyaux fourchus 10 et en embout nasal 11 ayant des dents 12,
**caractérisé en ce que**
le connecteur d'applicateur 7 comprend une chambre de pression 21 qui a une interface d'humidificateur 17 pour la connexion à un dispositif de thérapie High-Flow 1, un port 13 d'alimentation en oxygène ayant un diamètre d'ouverture d'au moins 1 mm, et un port 14 d'alimentation en air thérapeutique pour le tuyau d'alimentation 8, l'interface d'humidificateur 17 et le port 13 d'alimentation en oxygène chacun ayant un siège de soupape 23 inférieur et un siège de soupape 25 supérieur dans la chambre de pression 21 qui sont munis d'un joint 22 et un corps de soupape 24 étant déplaçable entre lesdits sièges de soupape 23, 25 et ledit corps de soupape 24 étant soumis à une force par un ressort 26 de compression hélicoïdal à partir de la direction du port 13 d'alimentation en oxygène et, de l'autre côté, étant poussé contre le siège de soupape 25 supérieur par un élément d'actionnement 28 du dispositif de thérapie High-Flow 1 quand le connecteur d'applicateur 7 est situé de manière arrêtée sur le dispositif de thérapie High-Flow 1.

2. Applicateur multifonctionnel 6 utilisable de manière mobile selon la revendication 1,
**caractérisé en ce que**
le corps de soupape 24 est une bille.

3. Applicateur multifonctionnel 6 utilisable de manière mobile selon la revendication 1,
**caractérisé en ce que**
le corps de soupape 24 est un piston de vérin.

4. Applicateur multifonctionnel 6 utilisable de manière mobile selon la revendication 1, 2 ou 3,
**caractérisé en ce que**
le connecteur d'applicateur 7 a des contacts 18, 19 pour une alimentation en courant et/ou pour des lignes de signaux et/ou de données.

5. Applicateur multifonctionnel 6 utilisable de manière mobile selon l'une quelconque des revendications précédentes,
**caractérisé en ce qu'**
une unité fonctionnelle 29 comprenant des capteurs 33 est intégrée dans le raccord en Y 9.

6. Applicateur multifonctionnel 6 utilisable de manière mobile selon l'une quelconque des revendications précédentes,
**caractérisé en ce qu'**
une unité fonctionnelle 29 ayant un distributeur 30 est intégrée dans le raccord en Y 9.

7. Applicateur multifonctionnel 6 utilisable de manière mobile selon l'une quelconque des revendications précédentes,
**caractérisé en ce qu'**
une unité fonctionnelle 29 ayant des émetteurs et des récepteurs qui communiquent avec des émetteurs et des receveurs dans le connecteur d'applicateur 7 ou dans le dispositif de thérapie High-Flow 1 est intégrée dans le raccord en Y 9.

8. Applicateur multifonctionnel 6 utilisable de manière mobile selon l'une quelconque des revendications précédentes,
**caractérisé en ce qu'**
une unité fonctionnelle 29 ayant une unité de traitement électronique 34 est intégrée dans le raccord en Y 9.

9. Applicateur multifonctionnel 6 utilisable de manière mobile selon la revendication 8,
**caractérisé en ce que**
l'unité de traitement électrique 34 est alimentée en énergie par l'intermédiaire des fils de chauffage 31 dans le tuyau d'alimentation 8 ou par une batterie ou par une batterie rechargeable.

10. Applicateur multifonctionnel 6 utilisable de manière mobile selon l'une quelconque des revendications précédentes,
**caractérisé en ce que**
le connecteur d'applicateur 7 a un microprocesseur programmable.

11. Applicateur multifonctionnel 6 utilisable de manière mobile selon la revendication 10,
**caractérisé en ce que**
des données de service, des données du patient et ses paramètres thérapeutiques individuels sont enregistrés dans la mémoire du microprocesseur programmable.

12. Applicateur multifonctionnel 6 utilisable de manière mobile selon l'une quelconque des revendications précédentes,
**caractérisé en ce que**
le raccord en Y 9 n'est pas disposé à moins de 5 mm des dents 12.

13. Applicateur multifonctionnel 6 utilisable de manière mobile selon l'une quelconque des revendications précédentes,
**caractérisé en ce que**
les matériaux utilisés sont munis d'un revêtement antibactérien.

14. Applicateur multifonctionnel 6 utilisable de manière mobile selon l'une quelconque des revendications précédentes,
**caractérisé en ce que**
lors de l'utilisation mobile, une petite source d'oxygène ou d'air thérapeutique facile à manier est connectée au port 13 d'alimentation en oxygène.
